Europäisches Patentamt

European Patent Office

Office européen des brevets

(19)

(11) Publication number: **0 270 316**
**A2**

# (12) EUROPEAN PATENT APPLICATION

(21) Application number: 87310504.3

(22) Date of filing: 27.11.87

(51) Int. Cl.⁴: **A61K 31/415** , A61K 31/62 ,
//(A61K31/415,31:405,31:19)

(30) Priority: 04.12.86 US 937765

(43) Date of publication of application:
08.06.88 Bulletin 88/23

(84) Designated Contracting States:
AT BE CH DE ES FR GB GR IT LI LU NL SE

(71) Applicant: PFIZER INC.
235 East 42nd Street
New York, N.Y. 10017(US)

(72) Inventor: McKie, James Edward, Jr.
Bill Hill Road
Lyme Connecticut(US)
Inventor: Potts, Russell Owen
73A Fenwood Drive
Old Saybrook Connecticut(US)

(74) Representative: Wood, David John et al
Pfizer Limited Ramsgate Road
Sandwich Kent CT13 9NJ(GB)

(54) Topical compositions comprising 1-substituted imidazoles and NSAIDs for treatment of acne.

(57) Topical acne compositions comprising from 1-10% w/w of a lipophilic 1-substituted imidazole antimicrobial agent and from 1-25% w/w of a non-steroidal antiinflammatory agent.

EP 0 270 316 A2

# TOPICAL COMPOSITIONS COMPRISING 1-SUBSTITUTED IMIDAZOLES AND NSAIDS FOR TREATMENT OF ACNE

This application relates to pharmaceutical compositions useful for the treatment of acne. More particularly, it relates to pharmaceutical compositions comprising a 1-substituted imidazole antimicrobial agent and a non-steroidal antiinflammatory agent and their use for topical treatment of acne in a subject suffering therefrom.

Common inflammatory acne, is a disease which affects a particular type of hair follicle in man known as the pilosebaceous follicle. Located principally on the face, neck, upper back, shoulders and upper arms, these follicles can become plugged with sebum and dead skin cells in certain predisposed individuals at the onset of puberty. In such individuals a number of these plugged follicles subsequently develop into inflammed lesions of varying degrees of severity such as papules, pustules and cysts. In extreme cases canalizing inflammed and infected areas appear often causing permanent disfiguring scars. The microorganism most frequently found in the sebaceous duct is Propionibacterium acnes, a microorganism which is able to cleave the triglycerides of the sebum with release of fatty acids which cause the irritation and inflammation.

In view of the multiple factors involved in the development of acne and its often traumatic impact upon its victims, a variety of agents, alone and in combination, have been reported as useful for topical application for treatment of acne.

Vanden Bossche et al., British Journal of Dermatology, 107, 343-348 (1982) and Dermatologica 164, 201-208 (1982) ; and Kavli et al ., Zeitschrift Hautkronkheit 59 (13) , 882-887 report that the combination of miconazole and benzoyl peroxide provides better topical treatment of acne than either agent alone.

U.S. Patent 4,497,794 describes a process and composition for topical treatment of acne which comprises erythromycin and certain derivatives thereof and certain organic peracids, especially benzoyl perioxide.

Wong et al., J. Am. Acad. Dermatol. 11 (6), 1076-1081 (1984) report the combination of tetracycline and ibuprofen, a non-steroidal antiinflammatory agent (NSAID) to be more effective for oral treatment of acne than either agent alone. More recently, Funt, J. Am. Acad. Dermatol. 13 (3) , 525-526 (1985) reported the successful use of minocycline plus ibuprofen for oral treatment of acne. Popovich, Pharmacy International, March 1986, pages 71-74, presents a summary of current therapy for acne vulgaris. He speculates that the Wong et al. publication ( loc. cit.) will encourage further research into combinations of NSAID agents and contemporary antiacne drugs.

The combination of miconazole and benzoyl peroxide for topical treatment of acne is reported in Chemist & Druggist, Sept. 8, 1984.

The oral use of imidazole antibiotics, especially ketoconazole and metronidazole, for treatment of psoriasis or seborrheic dermatitis in humans is described in U.S. Patent 4,491,588, issued January 1, 1985. U.S. Patent 4,569,935, issued February 11, 1986, describes topical application of an imidazole antibiotic, especially ketoconazole and clotrimazole, for treatment of psoriasis and seborrheic dermatitis in humans.

Ghetti et al., Arch. Dermatol. 122, 629 (1986) report on the effective oral use of ketoconazole in the treatment of acne in women, and note further studies are required to assess its effectiveness as a topical agent.

Akhter et al., J. Pharm. Phamacol. 37, 27-37 (1985) report on the absorption of ibuprofen and flurbiprofen through human skin.

Combinations of benzoyl peroxide and an antimicrobial agent are subject to instability and/or irritation. Orally administered prior art compositions are frequently accompanied by gastrointestinal side effects. None of the prior art compositions has been completely satisfactory for treatment of acne and improved compositions for said treatment are desirable.

The present invention comprises compositions having enhanced efficacy for topical treatment of acne. The compositions comprise a 1-substituted imidazole antimicrobial agent and a non-steroidal antiinflammatory agent (NSAID). It also comprises the use of the compositions for topical treatment of acne in a subject suffering therefrom by treating the site of the acne with an anti-acne effective amount of said composition.

The 1-substituted imidazoles useful in the compositions of this invention are the antimicrobial, especially antifungal, imidazoles described in the following references:

U.S. Patent 4,062,966, issued December 13, 1977 which describes a variety of 1-aryl-2-(1-imidazolyl)-alkyl ethers and thioethers;

U.S. Patent 3,705,172, issued December 5, 1972, which discloses triarylmethyl substituted imidazoles;

U.S. Patent 3,717,655, issued February 20, 1973 describing 1-( β -aryl)ethylimidazole ethers;

U.S. Patent 4,144,346, issued March 13, 1979, which describes 1-(1,3-dioxolan-2-ylmethyl)-1H-imidazoles;

and the wide variety of antifungal 1-substituted imidazoles enumerated in the excellent bibliography of such compounds presented in U.S. Patent 4,277,475, issued July 7, 1981, all of which are incorporated herein by reference.

The favored 1-substituted imidazoles are those having sufficient lipophilic character to permit their penetration to and concentration in the oil-rich follicular environment at a level sufficient for them to exhibit activity against P. acnes, especially in the presence of a NSAID, a substance which tends to enhance the activity of the 1-substituted imidazole and even to exhibit a synergistic effect.

This property is readily measurable by methods known to those skilled in the art such as, for example, skin layer analysis which measures the amount of radiolabeled drug, e.g. tioconazole, in human skin treated with compositions containing the drug. [Ekerdt et al., Brit. J. Dermatol. 115, Suppl. 31, 36-40 (1986)]. Frozen section autoradiography of human skin treated with radiolabeled tioconazole confirmed that tioconazole was localized in the sebaceous follicle.

Preferred 1-substituted imidazoles in the compositions of this invention are:

clotrimazole: 1-[(2-chlorophenyl)diphenylmethyl]-1H-imidazole;

econazole: 1-[2-[(4-chlorophenyl)methoxy]-2-(2,4-dichlorophenyl)ethyl]-1H-imidazole;

ketoconazole: 1-acetyl-4-[4-[[2-(2,4-dichlorophenyl)-2-(1H-imidazol-1-ylmethyl)-1,3-dioxolan-4-yl]-methoxy]phenyl]piperazine;

miconazole: 1-[2-(2,4-dichlorophenyl)-2-[2,4-dichlorophenyl)methoxy]ethyl]-1H-imidazole; and

tioconazole: 1-[2-[(2-chloro-3-thienyl)methoxy]-2-(2,4-dichlorophenyl)ethyl]-1H-imidazole.

The NSAIDs useful in this invention are those which have sufficient lipophilicity to penetrate to and to concentrate in the oil-rich follicular site, particularly in the presence of a 1-substituted imidazole, and especially in the presence of a preferred 1-substituted imidazole enumerated above. Said substances tend to enhance and even synergize their antiinflammatory activity. Here also, the lipophilicity of the NSAIDs is measured by methods known to those skilled in the art.

Representative NSAIDs are those belonging to the classes of antiinflammatory agents such as the substituted benzoic acids, heterocyclic substituted acetic acids, phenoxybenzene acetic acids, phenylaminobenzene acetic acids, oxicams and aryl substituted propanoic acids.

Preferred NSAIDs within the enumerated classes are the following:

Aspirin: 2-(acetyloxy)benzoic acid;

Carprofen: 6-chloro-alpha-methyl-9H-carbazole-2-acetic acid;

Diclofenac Sodium: 2-[(2,6-dichlorophenyl)amino]-benzeneacetic acid monosodium salt;

Diflusinal: 2',4'-difluoro-4-hydroxy-[1,1'-biphenyl]-3-carboxylic acid;

Etodolac: 1,8-diethyl-1,3,4,9-tetrahydro-pyramo-[3,4-b]indole-1-acetic acid;

Fenoprofen: alpha-methyl-3-phenoxybenzeneacetic acid;

Flufenamic Acid: 2-[[3-(trifluoromethyl)phenyl]-amino]benzoic acid;

Flurbiprofen: 2-fluoro-alpha-methyl[1,1'-biphenyl]-4-acetic acid;

Ibufenac: 4-(2-methylpropyl)benzeneacetic acid;

Ibuprofen: alpha-methyl-4-(2-methylpropyl)-benzeneacetic acid;

Indomethacin: 1-(4-chlorobenzoyl)-5-methoxy-2-methyl-1H-indole-3-acetic acid;

Isoxicam: 4-hydroxy-2-methyl-N-(5-methyl-3-isoxazolyl)-2H-1,2-benzothiazone-3-carboxamide 1,1-dioxide;

Ketoprofen: 3-benzoyl-alpha-methylbenzeneacetic acid;

Meclofenamic Acid : 2-[(2,6-dichloro-3-methylphenyl)amino]benzoic acid;

Naproxen: 6-methoxy-alpha-methyl-2-naphthaleneacetic acid;

Oxaprozin: 4,5-diphenyl-2-oxazolipropanoic acid;

Piroxicam: 4-hydroxy-2-methyl-N-2-pyridinyl-2H-1,2-benzothiazine-3-carboxamide 1,1-dioxide;

Pranoprofen: alpha-methyl-5H-[1]benzopyrano-[2,3-b]pyridine-7-acetic acid;

Sulindac: cis-S-fluoro-2-methyl-1-[(p-methylsulfinyl)benzylidene]indene-3-acetic acid;

Suprofen: alpha-methyl-4-(2-thienylcarbonyl)-benzeneacetic acid;

Tenoxicam: 4-hydroxy-2-methyl-N-2-pyridinyl-2H-thieno[2,3-e]-1,2-thiazine-3-carboxamide 1,1-dioxide;

Tolmetin: 1-methyl-5-(4-methylbenzoyl)-1H-pyrrole-2-acetic acid; and

Zomepirac: 5-(4-chlorobenzoyl)-1,4-dimethyl-1H-pyrrole-2-acetic acid.

While the focus of this invention is the topical administration of compositions comprising a 1-substituted imidazole and a NSAID in a suitable vehicle, the individual ingredients; i.e., imidazole and NSAID, can be separately applied to the skin. If this mode of administration is adopted, the 1-substituted imidazole is generally applied first, followed immediately or soon thereafter, by the NSAID. It is, however, preferred to apply a mixture of the two ingredients since a composition comprising both permits consistent application of the ingredients in proper amounts of each.

The 1-substituted imidazole components of the compositions of this invention, all known antifungal agents, also exhibit antibacterial activity. Tioconazole, for example, has been found to exhibit activity against P. acnes; an average minimum inhibitory concentration (MIC) of 5 mcg./ml. being exhibited against a population of 12 P. acnes strains.

Suitable vehicles for the topical compositions of this invention comprising a 1-substituted imidazole and a NSAID are known to those skilled in the art. Representative vehicles are set forth herein in the examples of suitable topical compositions comprising a 1-substituted imidazole and a NSAID. Typical topical compositions may comprise a surfactant, a debridging agent, a fungicide-bactericide, oils, fats, waxes, a cationic emollient and/or emollient esters, organic solvents, water, and, when a gel is desired, a gelling agent. The compositions can be in the form of gel, a cream or a solution. Preparation of pharmaceutically elegant compositions is facilitated by the use of finely divided particles of the imidazole and NSAID ingredients. Desirably, micronized particles of said ingredients of less than about 150 microns are used.

The compositions of this invention comprise from 1-10% w/w of a 1-substituted imidazole and from 1-25% w/w of a NSAID. The favored amounts of imidazole agent range from 1-5% w/w and the preferred amounts from 1-2% w/w. The favored amounts of the NSAID range from 1-15% w/w and the preferred amounts from 5-10% w/w. In general, topical application of the herein described compositions from 2 to 4 times a day achieves marked improvement of the acne affliction. The compositions are gently applied with light rubbing to achieve uniform distribution of the composition.

The following examples illustrate representative compositions of this invention. They are not intended to be limiting thereof since, as those skilled in the art will recognize, many variations of the compositions, and particularly of the vehicles exemplified, are possible.

## EXAMPLE 1

| Ingredients | % w/w |
|---|---|
| Ibuprofen | 10.00 |
| Tioconazole | 1.00 |
| Polyethylene Glycol 300 | 10.00 |
| Carbomer 934P[a] | 1.00 |
| Triethanolamine 99% | 1.35 |
| Phenoxyethanol | 1.00 |
| Purified Water          q.s. to | 100.00 |

[a] polymer of acrylic acid cross-linked with a polyfunctional agent; available from B. F. Goodrich Chemical Co., 6100 Oak Tree Blvd., Cleveland, Ohio 44131.

The ibuprofen and tioconazole (actives) were suspended in the polyethylene glycol 300 and a portion of the water. The Carbomer 934P was then dispersed in water and complete hydration achieved by heating the water to 80°C.-85°C. The dispersion was neutralized with triethanolamine and cooled. Then, the phenoxyethanol was added followed by the slurry of actives and the mixture homogenized to a smooth, white gel.

4

## EXAMPLE 2

| Ingredients | % w/w |
|---|---|
| Ibuprofen | 10.00 |
| Tioconazole | 1.00 |
| Dimethyl Isosorbide | 15.00 |
| Propylene Glycol | 7.00 |
| Polymer JR-400 [a] | 7.00 |
| Necon LO [b] | 0.10 |
| Arlacel 165 [c] | 0.05 |
| Allantoin | 1.50 |
| Methyl Paraben | 0.20 |
| Propyl Paraben | 0.20 |
| Purified Water | q.s. to 100.00 |

The ibuprofen and tioconazole (actives) were dissolved in a mixture of dimethyl isosorbide and propylene glycol. To purified water at 70°C.-75°C. was added the methyl and propyl parabens, Arlacel 165, and the Necon LO, followed by Polymer JR-400 and the allantoin. The aqueous solution was cooled and the solution containing the actives added thereto with a good mixing. Homogenization gave a smooth, white gel.

[a] cationic cellulosic resin; available from Union Carbide Corp., 1 Ridgebury Road, Danbury, CT 06817.

[b] dimethyl lauramine oleate; available from Alzo, Inc., 6 Gulfstream Blvd., Matawan, NJ.

[c] non-ionic fatty acid partial ester of a polyol anhydride; available from ICI, United States Inc.

## EXAMPLE 3

| | Ingredients | % w/w |
|---|---|---|
| Oil Phase | Cetyl Alcohol | 6.000 |
| | Glyceryl Monostearate | 3.000 |
| | Arachidyl Propionate | 2.000 |
| | Propyl Paraben | 0.200 |
| | Span 60 | 0.412 |
| Aqueous Phase | Dimethyl Isosorbide | 8.000 |
| | Propylene Glycol | 7.000 |
| | Necon CPS [a] | 0.150 |
| | Allantoin | 1.500 |
| | Brij 721 [b] | 1.584 |
| | Ibuprofen | 10.000 |
| | Tioconazole | 1.000 |
| | Purified Water | q.s. to 100.00 |

The cetyl alcohol, glycerylmonostearate, arachidyl propionate, and Span 60 were melted together at a temperature between 70°C.-75°C. The ibuprofen and tioconazole were dissolved in the mixture of dimethyl isosorbide and propylene glycol. The methyl paraben and Brij 721 were dissolved in hot water and the Necon CPS added, the temperature being held between 70°C.-75°C. The two phases were emulsifed and the allantoin added thereto with good mixing. The emulsion was cooled and the ibuprofen/tioconazole solution added. Homogenization afforded a smooth white cream.

[a] polyoxyethylene-15 cocamine phosphate/oleate complex; available from Alzo, Inc., 6 Gulfstream Blvd., Matawan, NJ.

[b] polyoxyethylene ether of higher aliphatic alcohols; available from ICI, United States, Inc.

## EXAMPLE 4

| | Ingredients | % w/w |
|---|---|---|
| Oil Phase | Cetyl Alcohol | 5.000 |
| | Glyceryl Monostearate | 3.000 |
| | Arachidyl Propionate | 2.000 |
| | Propyl Paraben | 0.200 |
| | Span 60 [a] | 0.454 |
| Aqueous Phase | Propylene Glycol | 7.000 |
| | Necon CPS | 0.150 |
| | Methyl Paraben | 0.200 |
| | Brij 721 | 1.546 |
| | Ibuprofen | 10.000 |
| | Tioconazole | 1.000 |
| | Purified Water    q.s. to | 100.000 |

The procedure of Example 3 was followed. Since no solvents (e.g., dimethylisosorbide, propylene glycol) were used, the ibuprofen and tioconazole were sprinkled into the emulsion. Homogenization gave a smooth white cream.

[a] fatty acid partial ester of sorbitol anhydride; available from ICI, United States, Inc.

EXAMPLE 5

The compositions tabulated below are prepared according to the procedures of the preceding examples but using the 1-substituted imidazoles and NSAIDs indicated.

| (a)<br>Imidazole | (b)<br>NSAID | Ratio<br>b:a | Proc. of<br>Example |
|---|---|---|---|
| tioconazole | ibuprofen | 5:1 | 1 |
| tioconazole | piroxicam | 10:1 | 2 |
| tioconazole | isoxicam | 10:1 | 2 |
| tioconazole | tenoxicam | 10:1 | 2 |
| tioconazole | aspirin | 8:1 | 1 |
| tioconzaole | fenoprofen | 25:1 | 2 |
| tioconazole | flufenamic acid | 25:10 | 3 |
| tioconazole | indomethacin | 10:4 | 3 |
| clotrimazole | ibuprofen | 10:1 | 1 |
| clotrimazole | piroxicam | 10:1 | 4 |
| clotrimazole | suprofen | 10:15 | 4 |
| clotrimazole | meclofenamic acid | 2:1 | 3 |
| clotrimazole | flurbiprofen | 10:6 | 3 |
| ketoconazole | isoxicam | 10:1 | 1 |
| ketoconazole | carprofen | 10:2 | 1 |
| ketoconazole | diclofenac sodium | 25:15 | 2 |
| ketoconazole | etodalac | 15:1 | 2 |
| ketoconazole | zomepirac | 12:1 | 2 |
| econazole | aspirin | 10:2 | 3 |
| econazole | diflunisal | 10:1 | 4 |
| econazole | ibufenac | 20:4 | 4 |

| (a) Imidazole | (b) NSAID | Ratio b:a | Proc. of Example |
|---|---|---|---|
| econazole | isoxicam | 20:2 | 3 |
| econazole | sulindac | 10:1 | 2 |
| econazole | ketoprofen | 10:1 | 2 |
| miconazole | ibuprofen | 10:1 | 1 |
| miconazole | piroxicam | 10:1 | 2 |
| miconazole | oxaprozin | 8:1 | 3 |
| miconazole | pranoprofen | 10:2 | 3 |
| miconazole | tolmetin | 15:5 | 3 |
| miconazole | naproxen | 20:8 | 4 |
| miconazole | indomethacin | 10:1 | 3 |
| miconazole | ibuprofen | 8:1 | 4 |

The above compositions are effective for topical treatment of acne.

**Claims**

1. A composition for the topical treatment of acne comprising from 1-10% w/w of the composition of a 1-substituted imidazole antimicrobial agent and from 1-25% w/w of the composition of a non-steroidal antiinflammatory agent.

2. A composition according to claim 1 wherein the 1-substituted imidazole is clotrimazole, econazole, ketoconazole, micronazole or tioconazole, and wherein the non-steroidal antiinflammatory agent is aspirin, carprofen, diclofenac sodium, diflunisal, etodolac, fenoprofen, flufenamic acid, flurbiprofen, ibufenac, ibuprofen, indomethacin, isoxicam, ketoprofen, meclofenamic acid, naproxen, oxaprozin, piroxicam, pranoprofen, sulindac, suprofen, tenoxicam, tolmetin or zomepirac.

3. A composition according to claim 2 wherein the 1-substituted imidazole is tioconazole, miconazole or ketoconazole and the antiinflammatory agent is aspirin, ibuprofen, indomethacin or piroxicam.

4. A composition according to claim 3 wherein the 1-substituted imidazole is tioconazole and the antiinflammatory agent is ibuprofen.

5. A composition according to claim 4 wherein the ratio of ibuprofen:tioconazole is about 10:1 w/w.

6. A composition according to any one of the preceding claims comprising from 1-5% w/w of the imidazole and from 1-15% w/w of the antiinflammatory agent.

7. A composition according to claim 6 which comprises from 1-2% w/w of the imidazole and from 5-10% w/w of the antiinflammatory agent.

8. The use of a 1-substituted imidazole antimicrobial agent and a non-steroidal antiinflammatory agent for the manufacture of a medicament for treating acne.

9. A use according to claim 8 wherein the medicament is a composition as claimed in any one of claims 1 to 7.

CLAIMS For the Contracting States: ES and GR

1. A method for making a composition for the topical treatment of acne which comprises combining from 1-10% w/w of the composition of a 1-substituted imidazole antimicrobial agent, from 1-25% w/w of the composition of a non-steroidal antiinflammatory agent, and a suitable vehicle.

2. A method according to claim 1 wherein the 1-substituted imidazole is clotrimazole, econazole, ketoconazole, miconazole or tioconazole, and wherein the non-steroidal antiinflammatory agent is aspirin, carprofen, diclofenac sodium, diflunisal, etodolac, fenoprofen, flufenamic acid, flurbiprofen, ibufenac, ibuprofen, indomethacin, isoxicam, ketoprofen, meclofenamic acid, naproxen, oxaprozin, piroxicam, pranoprofen, sulindac, suprofen, tenoxicam, tolmetin or zomepirac.

3. A method according to claim 2 wherein the 1-substituted imidazole is tioconazole, miconazole or ketoconazole and the antiinflammatory agent is aspirin, ibuprofen, indomethacin or piroxicam.

4. A method according to claim 3 wherein the 1-substituted imidazole is tioconazole and the antiinflammatory agent is ibuprofen.

5. A method according to claim 5 wherein the ratio of ibuprofen:tioconazole is 10:1 (w/w).

6. A method according to any one of the preceding claims wherein a composition comprising from 1-5% w/w of the imidazole and from 1-15% w/w of the antiinflammatory agent is prepared.

7. A method according to claim 6, wherein a composition comprising from 1-2% w/w of the imidazole and from 5-10% w/w of the antiinflammatory agent is prepared.

8. The use of a 1-substituted imidazole antimicrobial agent and a non-steroidal antiinflammatory agent for the manufacture of a medicament for treating acne.

9. The use of a 1-substituted imidazole antimicrobial agent and a non-steroidal antiinflammatory agent for the manufacture of a medicament for the topical treatment of acne in which medicament the imidazole is present in an amount of from 1-10% w/w and the antiinflammatory agent from 1-25% w/w.